# EUROPÄISCHE PATENTSCHRIFT

(11) **EP 0 600 949 B1**
(45) Veröffentlichungstag und Bekanntmachung des Hinweises auf die Patenterteilung: **10.01.1996**
(21) Anmeldenummer: 92917133.8
(22) Anmeldetag: 10.08.1992
(51) Int. Cl.: C07C 233/22, A61K 31/165, C07C 235/34, C07C 233/29, C07C 275/28, C07D 209/26, C07D 213/82, C07C 237/20

(54) **NEUE 3,5-DI-TERT.BUTYL-4-HYDROXYPHENYL-DERIVATE, VERFAHREN ZU IHRER HERSTELLUNG UND ARZNEIMITTEL**
NEW 3,5-DI-TERT.BUTYL-4-HYDROXYPHENYL DERIVATIVES, METHODS OF PREPARING THEM AND DRUGS CONTAINING THEM
NOUVEAUX DERIVES 3,5-DI-TERT.BUTYL-4-HYDROXYPHENYLES, LEUR PROCEDE DE FABRICATION ET MEDICAMENTS LES RENFERMANT

(30) Priorität: 13.08.1991 DE 4126662
(43) Veröffentlichungstag der Anmeldung: 15.06.1994
(73) Patentinhaber: BOEHRINGER MANNHEIM GMBH, D-68298 Mannheim (DE)
(72) Erfinder: DRECKMANN-BEHRENDT, Bruno, D-6800 Mannheim 1 (DE); HECK, Reinhard, D-6718 Grünstadt 3 (DE); DRESEL, Alois, D-6905 Schriesheim (DE); MICHEL, Helmut, D-6800 Mannheim 31 (DE)
(74) Vertreter: Mink, Reinhold, Dr.
(86) Internationale Anmeldenummer: EP9201821
(87) Internationale Veröffentlichungsnummer: WO9304035

(56) Entgegenhaltungen:
- EP-A- 0 404 039
- EP-A- 0 405 233
- EP-A- 0 407 200
- EP-A- 0 415 123

## Beschreibung

Die vorliegende Erfindung betrifft neue Di-tert.-butyl-hydroxyphenyl-derivate (BHT-Derivate) der allgemeinen Formel I 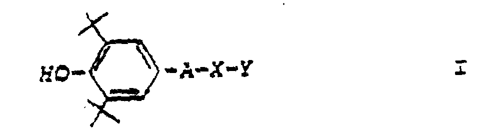 in der
- A: eine gerade oder eine verzweigte Alkylkette mit 1 bis 5 C-Atomen
- X: eine -NR-CO- (Amid) oder eine -NR-CO-NR-(Harnstoff) Gruppe, in der R ein Wasserstoffatom oder einen C₁-C₄-Alkylrest darstellt,
und
- Y: eine geradkettige oder verzweigte, gesättigte oder ungesättigte Kohlenwasserstoffkette mit 1 bis 6 C-Atomen, die durch einen Aryl-, Hetaryl- Aryloxy-, oder Arylthio-rest substituiert sein kann, einen C₃-C₆-Cycloalkyl-, oder einen Arylrest darstellt, wobei der betreffende Aryl- bzw. Hetarylrest jeweils ein bis dreifach in allen möglichen Positionen am Ring durch CN, Hydroxymethyl, Methylendioxy-, Halogen, Tri-fluormethyl, C₁-C₄-Alkyl, Amino, C₁-C₄-Acylamino, Di-(C₁-C₄,)-alkylamino, C₁-C₄-Alkoxy, Carboxyl, Oxy-essigsäureethylester oder Nitro subsitutiert sein kann,
bedeuten,
mit der Maßgabe, daß Y nur unsubstituiertes Phenyl bedeutet, wenn A eine andere Bedeutung als -CH₂- oder -CH₂-CH₂- hat,
sowie deren pharmakologische unbedenkliche Salze, Verfahren zu ihrer Herstellung und Arzneimittel, die diese Verbindungen enthalten.
Als "Brücke" A steht bevorzugt, die -CH₂-, -CH₂-CH₂-, -(CH₂)₃-, 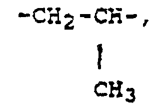 bzw. die -CH₂-C(CH₃)₂-Gruppe.

In der Carbonamidgruppe, in welcher der Hydroxyphenylrest über die "Brücke" A mit dem Amidstickstoff verbunden ist, bzw. in der Harnstoffgruppierung X stellt R bevorzugt ein Wasserstoffatom bzw. eine Methylgruppe dar.

Für Y ist der Benzyl, Phenethyl, Styryl-, der Phenyl und der Naphthylrest bevorzugt, deren Aromaten ihrerseits in allen Positionen ein bis dreifach, bevorzugt durch CN, Fluor, Chlor, Hydroxymethyl, Methyl, Trifluormethyl, Methoxy, Nitro, Dimethylamino, Methylendioxy, Oxyessigsäureethylester, Carboxyl, 2-Pyridyl, 3-Pyridyl, 4-Pyridyl, Pyridazyl, Pyrimidinyl, Pyrazyl oder 2-Indolyl substituiert sein kann. Als bevorzugter Hetarylrest gilt der 2-Furyl, 2-Thienyl, 2-Pyrryl, 2-Pyridyl, sowie der 3-Indolylrest.

Strukturell analoge Verbindungen finden als Zusätze bei Polymerisationsreaktionen Anwendung (vgl, U.S. -Pat. 4,152,319, U.S.-Pat. 3,780,103)

Als antihyperlipidaemisch ist 3,5-Di-tert.butyl-4-hydroxyacetanilid (ZA 7401080) bekannt.

Ebenso sind die konstitutionsisomeren Amide in dem EP-A 407 200 als Antihyperlipidaemica beschrieben.

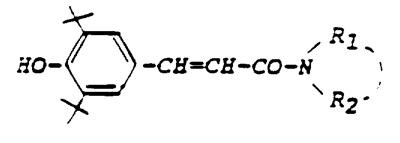

Weitere antioxidativ wirkende Verbindungen sind in dem EP-A 407 200 beschrieben.

Bekannt ist bereits das N-(3,5-Di-tert.-butyl-4-hydroxy)phenethyl-benzoesäureamid, J.Am.Chem.Soc. 84, 1629 (1962), jedoch sind in dieser Arbeit nur physikalisch-chemische Eigenschaften der genannten Verbindung beschrieben.

Verbindungen mit zwei BHT-Resten, die ebenfalls antioxidativ wirken, sind in EP-A 404 039 beschrieben.

In einer russischen Arbeit ist der N-(3,5-Di-tert.butyl-4-hydroxy)methyl-N'-phenylharnstoff als Treibstoffzusatz beschrieben, Nefthekhimiya 27 (5), 703.

Die Verbindungen I der vorliegenden Erfindung verfügen über eine starke Antioxidantienaktivität. Die Lipophilie dieser Antioxidantiengruppe bedingt, daß die Verbindungen sich im atherogenen low-density lipoprotein (LDL) anreichern und die sensitiven Bestandteile des LDL wirksam gegen reaktive Sauerstoffspezies schützen. Damit wird aber der LDL-Influx in die makrophagozytaren Schaumzellen deutlich abgebremst; denn die pathologisch gesteigerte Aufnahme von atherogenem LDL in den Atheromzellen setzt dessen oxidative Modifikation voraus.

Antioxidantien sind Substanzen, die - allgemein gesehen - eine erhebliche Verzögerung der oxidativen Vorgänge bei einem zu schützenden Produkt bewirken. Ein potent antiatherosklerotisch wirksames Antioxidans ist Probucol ^{R}, das eine hypolipidämische Wirkung bei verschiedenen Tierspezies und am Menschen hat. Es ist ein sterisch gehindertes Alkylphenol, das im LDL akkumuliert. Im Tierversuch wurde gezeigt, daß Probucol die oxidative Modifikation von LDL in der Arterienwand blockiert und die Atherombildung direkt aufgrund der Antioxidansaktivität verhindert (D. Steinberg et al., Amer. J. Cardiol. 57, 16 M (1986).

Nachteile von Probucol^{R} liegen in der geringen Resorption der Substanz, sowie in der extrem langen Verweildauer im Körpergewebe; die Ausscheidung von Probucol erfolgt hauptsächlich über die Faeces (vgl. M.N. Cayen, Pharmacol. Ther. 29, 157 (1985)).

Ferner senken Verbindungen der Formel I die Plasmalipide indem sie die intestinale Cholesterinresorption blockieren, in Konsequenz den intrahepatischen Pool des freien Cholesterins reduzieren und die Sektretion der nahrungsabhängigen Lipoproteine aus der Leber in das Plasma entsprechend vermindern. Die Inhibition der Cholesterinresporption beruht auf einer Hemmung der Acyl-Coenzym A: -cholesterintransferase (ACAT) Reaktion. In den Enterozyten katalysiert ACAT die Veresterung des Cholesterins, die notwendig ist, um Cholesterin in Chylomikronen einzupacken und über Darmlymphe und den Ductus thoracicus in das zirkulierende Blut einzuführen.

Die Substanzen verfügen über eine gute Resorption und inhibieren die ACAT abhängige Veresterung des freien Cholesterins nicht nur in den Enterozyten, sondern auch in den Zellen des Atheroms selbst. Sie verhindern dadurch deren schaumzellige Degeneration infolge Überladung mit Cholesterinester.

Di-tert.butyl-Phenolderivate der Formel I können nach an sich bekannten Syntheseverfahren erhalten werden, beispielsweise durch Umsetzung eines Amins der Formel II mit einer Carbonsäure, bzw. eines Derivats einer Carbonsäure der allgemeinen Formel III 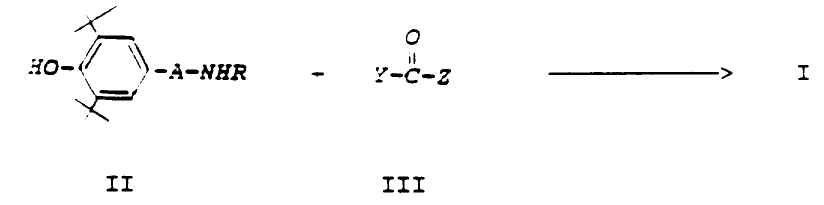 in denen A, R und Y die in Formel I angegebene Bedeutung haben und Z Hydroxyl, Halogen, C₁-C₄-Alkoxy oder Azid darstellt.

Die Umsetzung erfolgt in der Regel in einem inerten Lösungsmittel, wie einem Kohlenwasserstoff, Dimethylformamid, Dichlormethan oder in Ether.

Vorzugsweise verwendet man äquimolare Mengen an Amin und Carbonsäure in Gegenwart äquimolarer Mengen einer Base wie einem tert. organ. Amin, bevorzugt Triethylamin, Alkalibicarbonat, -Hydroxyd oder - Carbonat.

Als Derivate der Carbonsäure (Formel III) können das Säurehalogenid, -azid, -anhydrid oder -ester dienen.

Für die Umsetzung der freien Carbonsäure ist in der Regel die Anwesenheit von POCl₃, SOCl₂ oder PCl₃ erforderlich für die Umsetzung eines Carbonsäure-Esters mit dem Amin ein basischer Katalysator, bevorzugt ein Alkalialkoholat. In vielen Fällen gelingt auch die Reaktion der Carbonsäure mit einem Amin in einem Lösungsmittel wie z.b. Xylol durch Erwärmen unter Rückfluß am Wasserabscheider.

Die Harnstoff-Derivate der allgem. Formel I (X = -NR-C-NR-) werden vorzugsweise durch Addition eines Isocyanats IV an ein Amin der Formel II, durchgeführt in einem inerten Lösungsmittel z.B. Toluol, erhalten (vgl. Houben-Weyl, Bd. VIII S. 157).

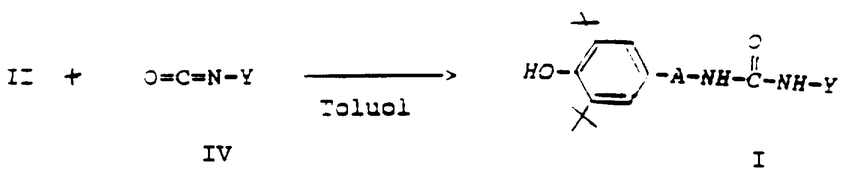

Die Verbindungen der Formel I finden aufgrund ihrer stabilisierenden Wirkung auf Lipoproteine Verwendung als Arzneimittel, insbesondere als Antiatherosclerotica.

Desweiteren wirken sie antiinflammatorisch, cytoprotektiv sowie antiasthmatisch. Sie können aber auch als Inhibitoren der reperfusionsabhängigen Lipidperoxidation und als Stabilisatoren des "lung surfactant factor" eingesetzt werden.

Falls einzelne Reaktionsprodukte nicht in genügender Reinheit anfallen, kann die Reinigung der Rohprodukte durch Kristallisation oder Säulenchromatographie erfolgen.

Zur Herstellung von Salzen mit physiologisch verträglichen organischen oder anorganischen Basen wie beispielsweise Natriumhydroxid, Kaliumhydroxid, Calciumhydroxid, Ammoniumhydroxid, Methylglucamin, Morpholin, Triethylamin oder Ethanolamin können die Verbindungen der Formel I mit den entsprechenden Basen umgesetzt werden. Auch Mischungen der sauren Verbindungen mit einem geeigneten Alkalicarbonat bzw.- hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Aussmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 10 mg/kg Körpergewicht.

Die nachfolgenden Beispiele zeigen einige Verfahrensvarianten, die zur Synthese der erfindungsgemäßen Verbindungen verwendet werden können. Sie sollen jedoch keine Einschränkung im Sinne des Erfindungsgedanken darstellen.

### Beispiel 1

### N-(3,5-Di-tert.butyl-4-hydroxybenzyl)-3-(4-chlorphenyl) acrylamid

Zu einer Lösung von 10.0 g (42 mmol) 3,5-Di-tert.butyl-4-hydroxybenzylamin. HCl (z.B. analog Houben-Weyl, Bd. 11/1, S. 502) und 5.4 ml Triethylamin in 100 ml absolutem Toluol tropft man 40 mol 4-Chlorzimtsäurechlorid (aus 7.3 g (40 mmol) 4-Chlorzimtsäure, analog Houben-Weyl Bd.8, S. 464) gelöst in 30 ml Toluol innerhalb 1 Stunde bei Raumtemperatur. Nach 14 Stunden Rühren gießt man den Ansatz in kalte 1 n Salzsäure, trennt die Phasen und schüttelt die organische Phase zweifach mit je 100 ml Wasser.
Nach Trocknen der Toluolphase über MgSO₄ wird das Lösungsmittel i.Vak. entfernt, der Rückstand mit wenig Ligroin angerieben und abgesaugt.
8.7 g fast farblose Kristalle, Fp: 175°C.

### Beispiel 2

### a) N-Methyl-[3.5-di-tert.butyl-4-hydroxy]phenylethylamin.HCI

Ein Gemisch aus 30.0 g (0.12 mol) 3,5-Di-tert.butyl-4-hydroxyphenethylamin und 13.4 ml (0.13 mol) Benzaldehyd in 200 ml absolutem Toluol wird am Wasserabscheider am Rückfluß erhitzt, bis sich kein Reaktionswasser mehr abscheidet. Anschließend engt man i.Vak. ein, löst erneut in 100 ml absolutem Toluol und versetzt diese Lösung mit 12.6 ml (0.13 mol) Dimethylsulfat. Nach 3 Stunden Erwärmen wird abgekühlt, 150 ml Wasser zugegeben, erneut 1 Stunde auf 120°C erwärmt und wieder abgekühlt. Die wässrige Phase wird abgetrennt, die organische Phase mit wenig HCl conc. versetzt und anschließen i.Vak. eingeengt. Noch vorhandenes Wasser wird mit Toluol azeotrop herausgeschleppt. Der verbleibende Rückstand wird mit wenig Ether/Ligroin verrieben und abgesaugt.
25.8 g bräunliche Kristalle, Fp: 200-203°C.

### b) N-2-(3,5-di-tert.butyl-4-hydroxy] phenyl)ethyl-N-methyl-3-(4-chlorphenyl)acrylamid

Ein Gemisch aus 4.0 g (15.6 mmol) des unter a) erhaltenen Amins, 2.6 ml (36 mmol) Pyridin und 100 ml Dichlormethan wird bei Raumtemperatur innerhalb 30 min mit einer Lösung von 16 mmol 4-Chlorzimtsäurechlorid (aus 2.9 g (16 mmol) 4-Chlorzimtsäure, analog Houben-Weyl Bd. 8, S. 464) in 20 ml Dichlormethan versetzt.

Nach 6 Stunden Rühren gießt man in kalte In HCl, trennt die Phasen und schüttelt die organische Phase zweifach mit Wasser aus. Nach Trocknen des Dichlormethans über Na₂SO₄ wird das Lösungsmittel i.Vak. entfernt. Nach Anreiben mit Ligroin/Toluol erhält man ein kristallines Produkt.
1.6 g farblose Kristalle, Fp: 156°C.

### Beispiel 3

### N-(3,5-Di-tert.butyl-4-hydroxybenzyl]-3-(3,4-methylendioxyphenyl)acrylamid

Zu einer Lösung von 2.7 g (10 mmol) 3.5-Di-tert.butyl-4-hydroxybenzylamin Hydrochlorid (hergestellt analog: Houben-Weyl, Bd. 11/1, S. 502 aus 3,5-Di-tert.butyl-4-hydroxybenzaldoxim) in 50 ml Tetrahydrofuran gibt man 4.2 ml Triethylamin und saugt nach 2 Stunden Rühren das gebildete Triethylamin Hydrochlorid ab. Zur Lösung der Base werden 2.2 g (10 mmol) 3,4-Methylendioxyphenyl-acrylsäureazid in 50 ml Tetrahydrofuran unter Eiskühlung zugetropft und über Nacht bei Raumtemperatur rühren lassen. Nach Eindampfen der Tetrahydrofuranlösung wird in Wasser und Essigester aufgenommen, trennt die organische Phase ab und schüttelt noch 2 mal mit Essigester. Nach Trocknen der Essigesterphase über Na₂SO₄ wird abdestilliert und der Rückstand mit Ether/Isohexan verrieben und abgesaugt. Es verbleiben 1.4 g farblose Kristalle, Fp. 194-197°C.

### Beispiel 4

### N-2-(3,5-Di-tert.butyl-4-hydroxyphenyl)ethyl-N'-phenyl-harnstoff

Eine Lösung von 2.0 g (8 mmol) 3,5-Di-tert.butyl-4-hydroxy-phenethylamin (z.B. nach J.Am.Chem.Soc. 84, 1629 (1962) in 30 ml Toluol wird tropfenweise mit 0.87 ml (8 mmol) Phenylisocyanat versetzt. Nach 2 Stunden Rühren wird das Lösungsmittel weitgehend entfernt, der Rückstand abgesaugt und aus Ethanol/Toluol (1:3) umkristallisiert.
2.8 g farblose Kristalle, Fp: 181-83°C.

### Beispiel 5

### N-(3,5-Di-tert.butyl-4-hydroxybenzyl)-3-(4-hydroxyphenyl)acrylamid

4.9 g (30 mmol) trans-4-Hydroxyzimtsäure werden in 50 ml wasserfreiem Aceton suspendiert und mit 12.6 ml (90 mmol) Triethylamin versetzt. Man kühlt im Eisbad auf etwa 5°C ab und tropft eine Lösung von 11.7 g (90 mol) Isobutylchloroformat in 12 ml Aceton zu. Nach 2 Stunden Rühren unter Eiskühlung wird eine Lösung von 6.3 g (96 mmol) Natriumazid in 24 ml Wasser zugetropft und noch weitere 2 Stunden ohne Kühlung weitergerührt. Man verdünnt mit etwa 300 ml Wasser und extrahiert das gebildete Azid mehrmals mit Essigester. Nach Eindampfen wird ein kristallines Produkt vom Fp. 74-76°C erhalten.

Das Azid wird fest zu 100 ml Tetrahydrofuranlösung von 20 mmol 3,5-Di-tert.butyl-4-hydroxybenzylamin (bereitet wie in Beispiel 3 beschrieben) eingetragen und 2 min bei Raumtemperatur gerührt. Durch Zugabe von 300 ml Wasser und Extrahieren mit Essigester erhält man 10 g der Isobutoxycarbonylverbindung. Durch Lösen mit 75 ml Tetrahydrofuran und Zugabe von 25 ml conc. Ammoniak wird der Ester verseift. Die Lösung wird abdestilliert in Wasser und Essigester gelöst, die organische Phase über Na₂so₄ getrocknet und im Vak. entfernt. Nach Anreiben und Absaugen erhält man 2.6 g fast farblose Kristalle, Fp. 205-208°C.

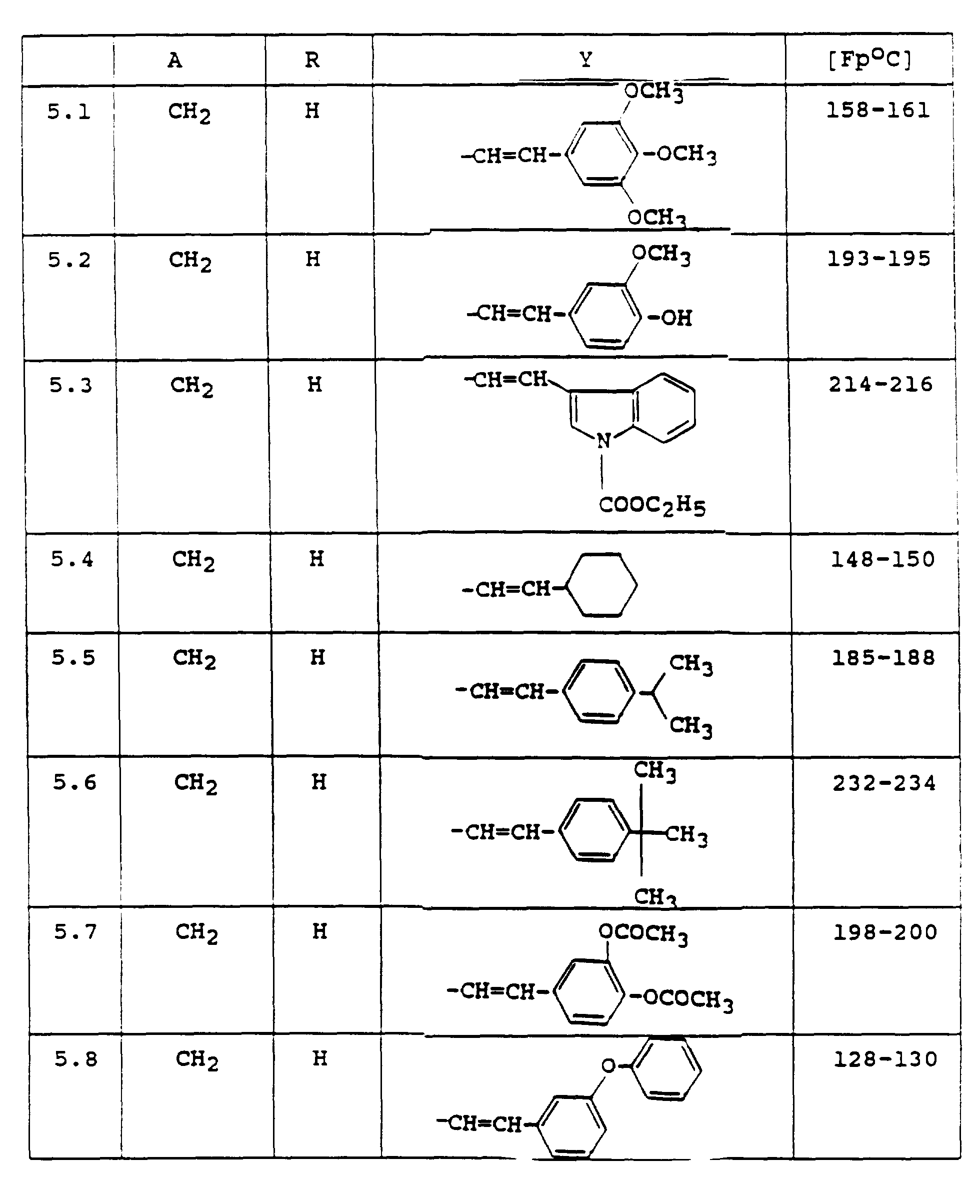

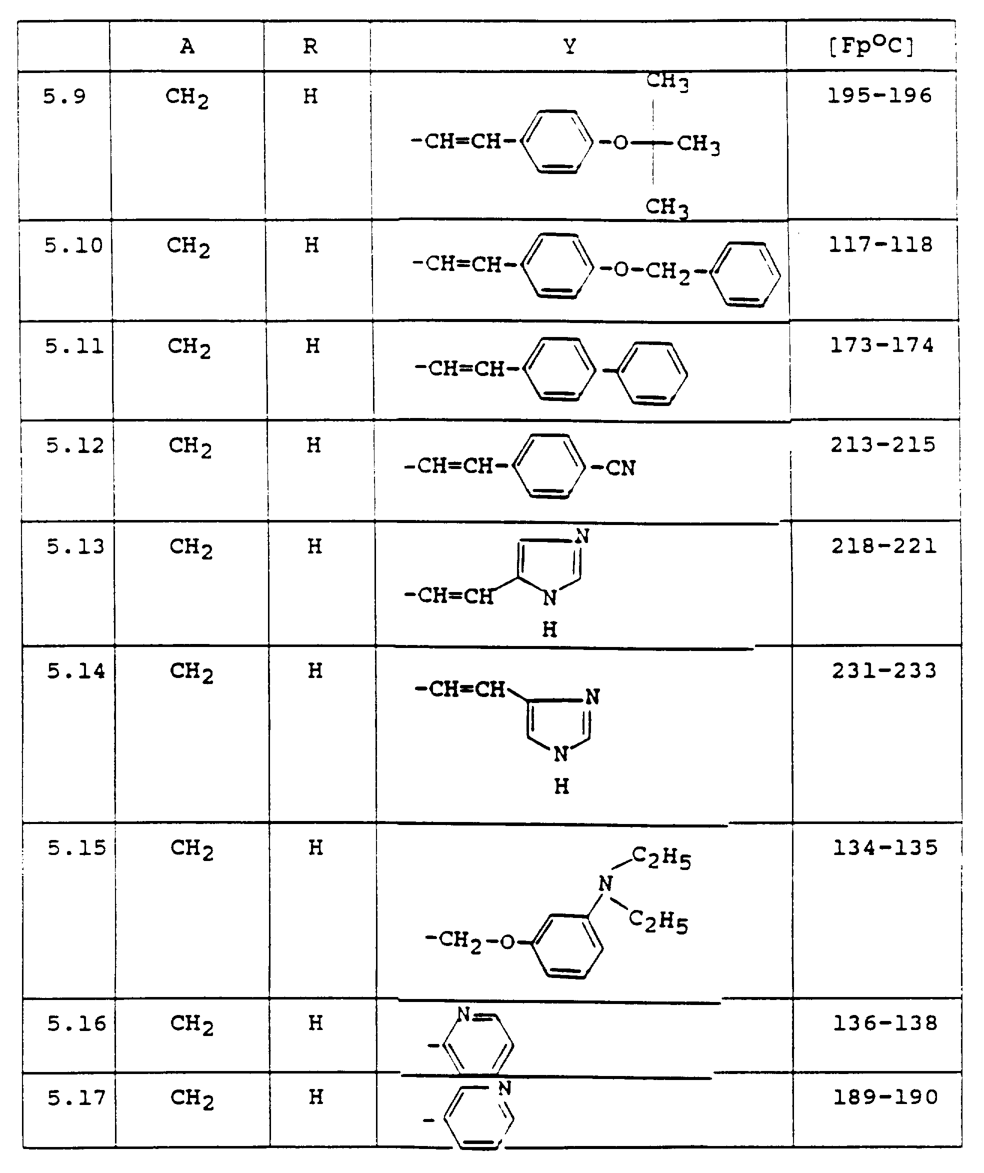

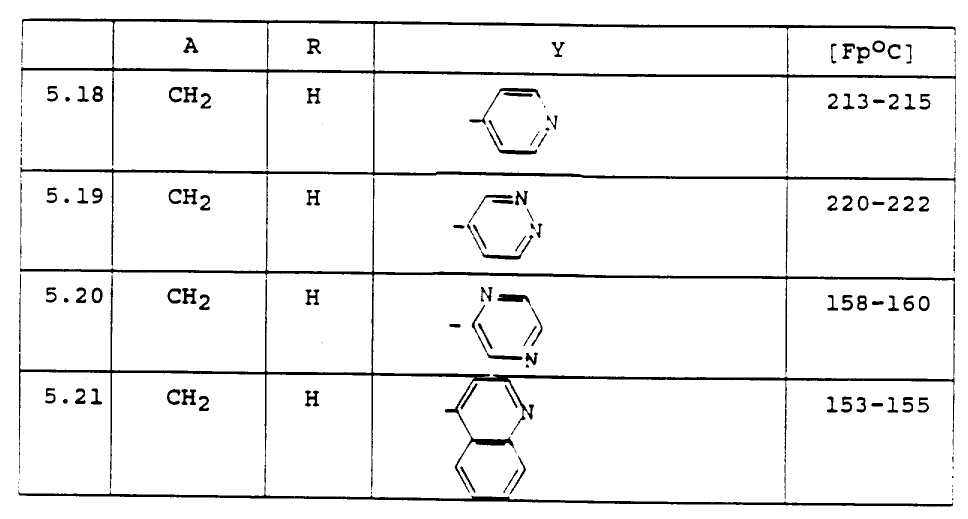

### Beispiel 6

### [4- [(3, 5-Di-tert.butyl-4-hydroxyphenyl)methyl]amino-(3-oxo-1-propenyl)phenoxy]essigsäureethylester

5.6 g (15 mmol) N-(3,5-Di-tert.butyl-4-hydroxybenzyl)-3-(4-hydroxyphenyl)acrylamid werden in 150 ml Aceton gelöst, 4.2 g Kaliumcarbonat zugegeben und mit 3.4 ml (30 mmol) Bromessigsäureethylester versetzt. Nach 24 Stunden Rühren bei Raumtemperatur wird i. Vak. eingeengt, in Wasser und Essigester gelöst, die Essigesterphase abgetrennt mit Na₂SO₄ getrocknet und abdestilliert. Der feste Rückstand wird mit Ether verrieben und abgesaugt. Es verbleiben 4.5 g farblose Kristalle, Fp. 144-147°C.

### Beispiel 7

### N-(3,5-Di-tert.butyl-4-hydroxybenzyl)-3-(4-dimethylaminophenyl)acrylamid

3.8 g (20 mmol) 4-Dimethylaminozimtsäure werden in 75 ml Tetrahydrofuran gelöst, 0.4 ml (60 mmol) Triethylamin zugegeben und unter Kühlung bei ca. 5°C 2.9 ml (22 mmol) Chlorameisensäureisobutylester zugetropft. Man läßt noch 1 Stunde im Eisbad und 1 Stunde bei Raumtemperatur nachrühren. Anschließend werden 2.8 g (10 mmol) 3,5-Di-tert.butyl-4-hydroxybenzylamin Hydrochlorid bei Raumtemperatur fest eingetragen und 24 Stunden weiter rühren lassen. Nach Zugabe von 300 ml Wasser wird 3 mal mit Essigester extrahiert. Die organische Phase mit Na₂SO₄ getrocknet und abdestilliert. Der verbleibende Rückstand wird über eine Kieselgel 60 Säule (60×600 mm) mit Isohexan/Essigester 1:1 gereinigt. Die Fraktion mit der reinen Verbindung werden abdestilliert, der Rückstand mit Ether verrieben und abgesaugt. Es verbleiben 2.6 g farblose Kristalle, Fp. 195-197°C.

### Beispiel 8

In analoger Weise wie in Beispiel 1 beschrieben werden die folgenden Verbindungen hergestellt:

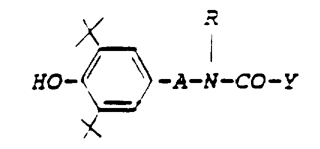

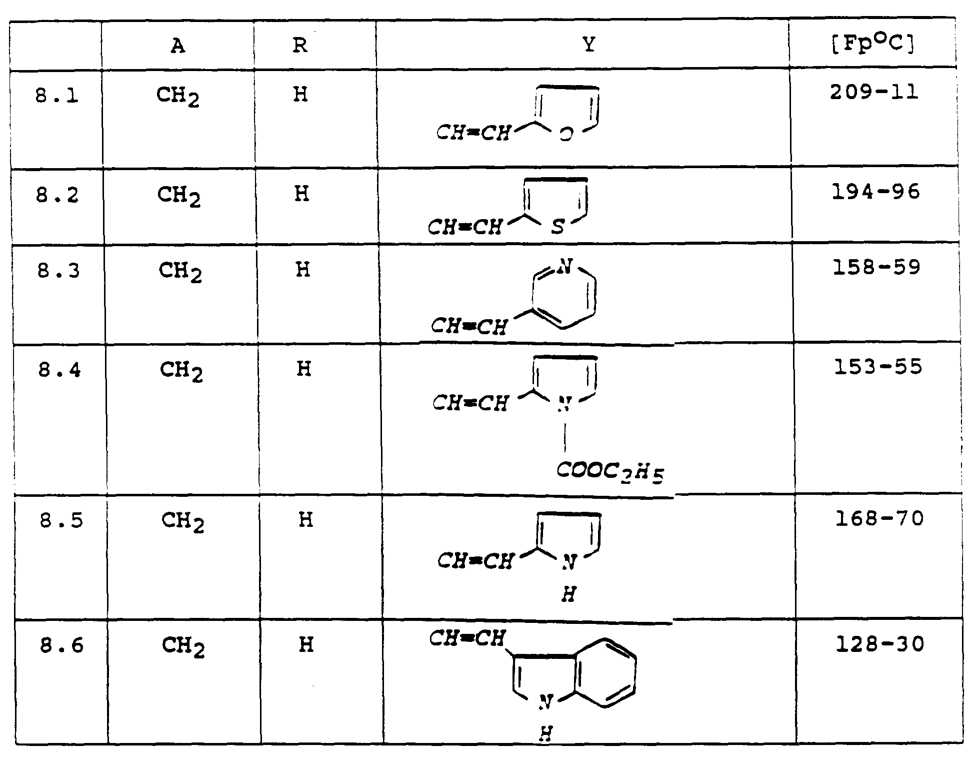

### Beispiel 9

In analoger Weise wie in Beispiel 4 beschrieben werden die folgenden Verbindungen hergestellt:

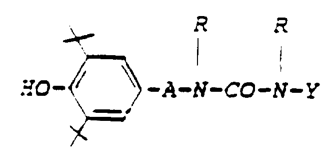

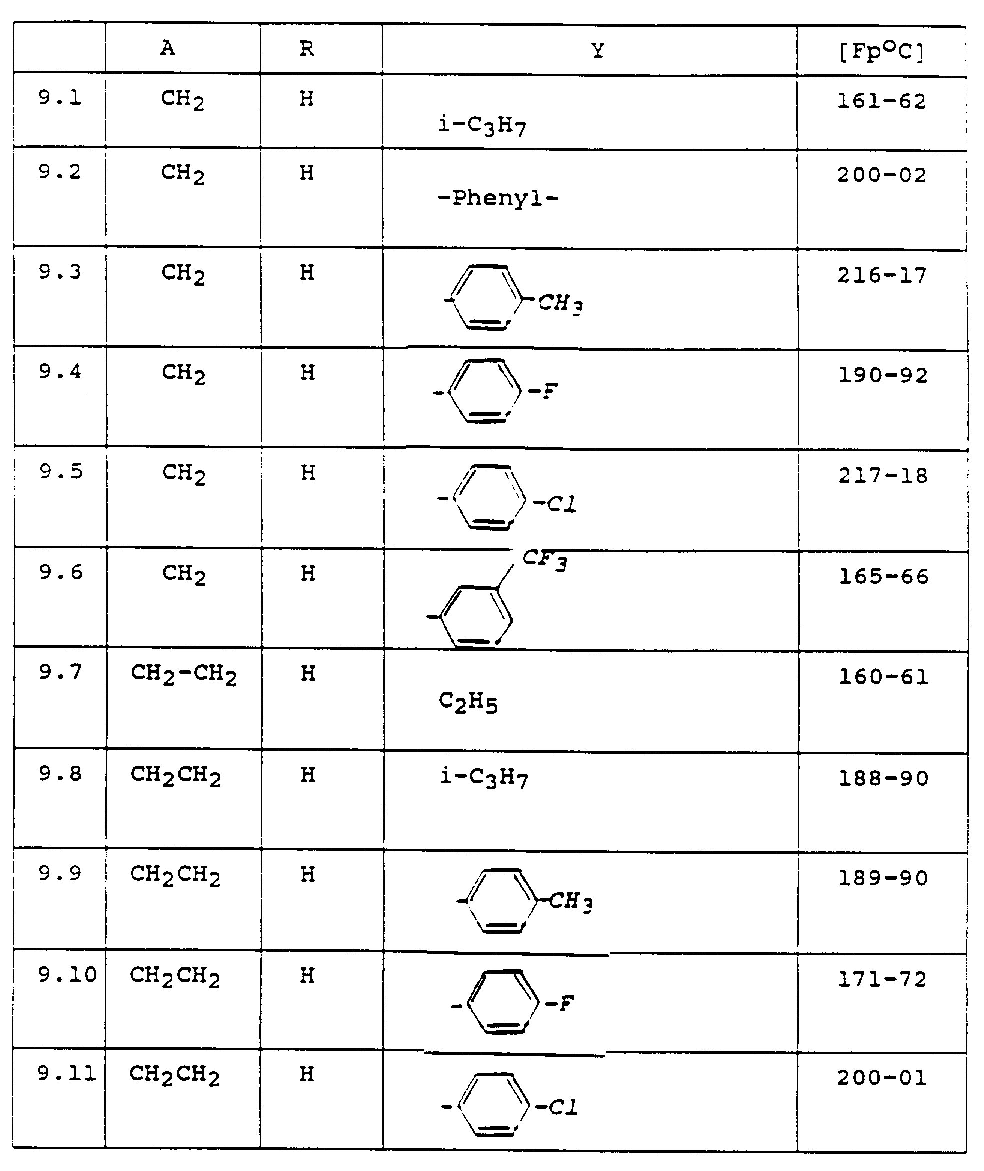

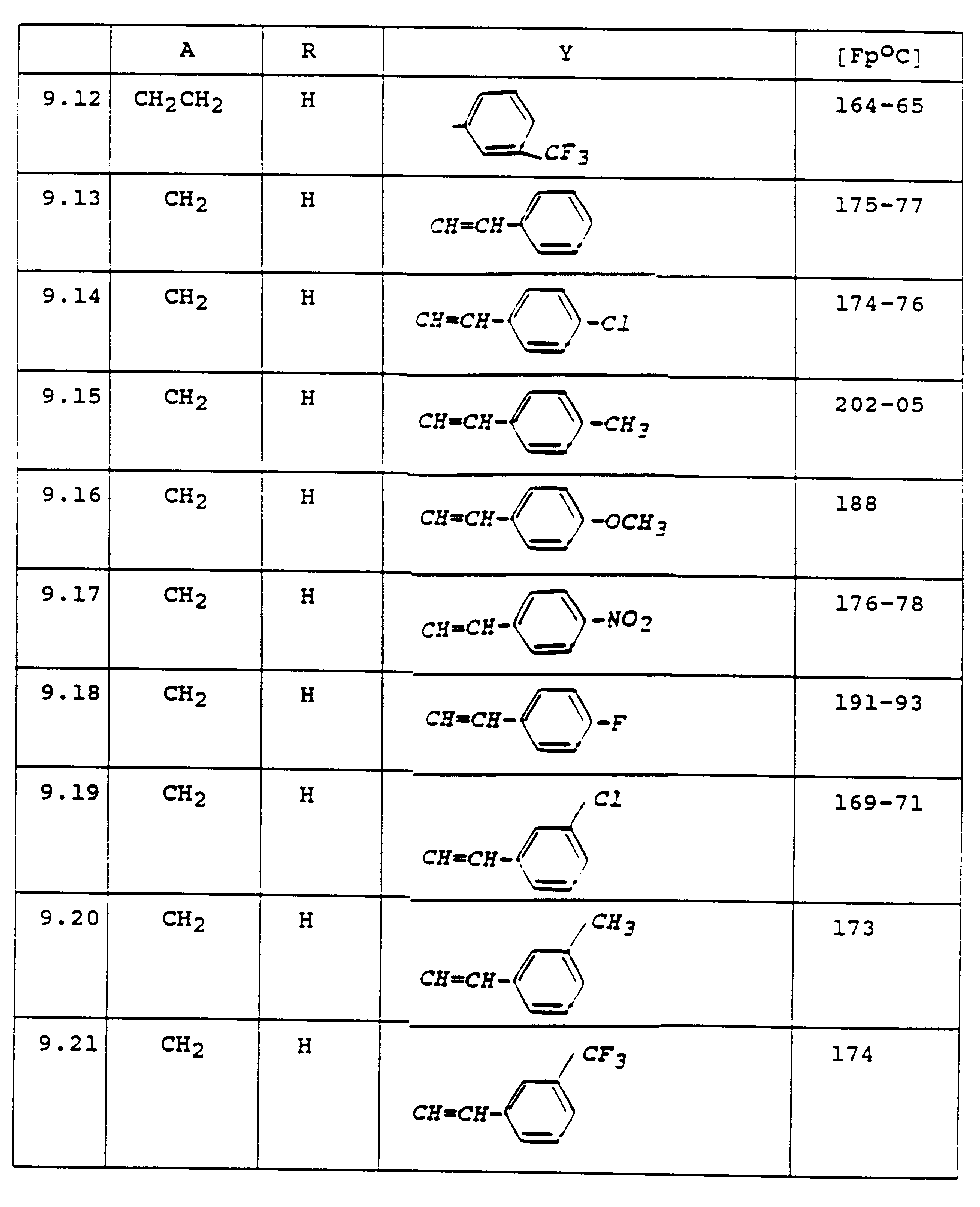

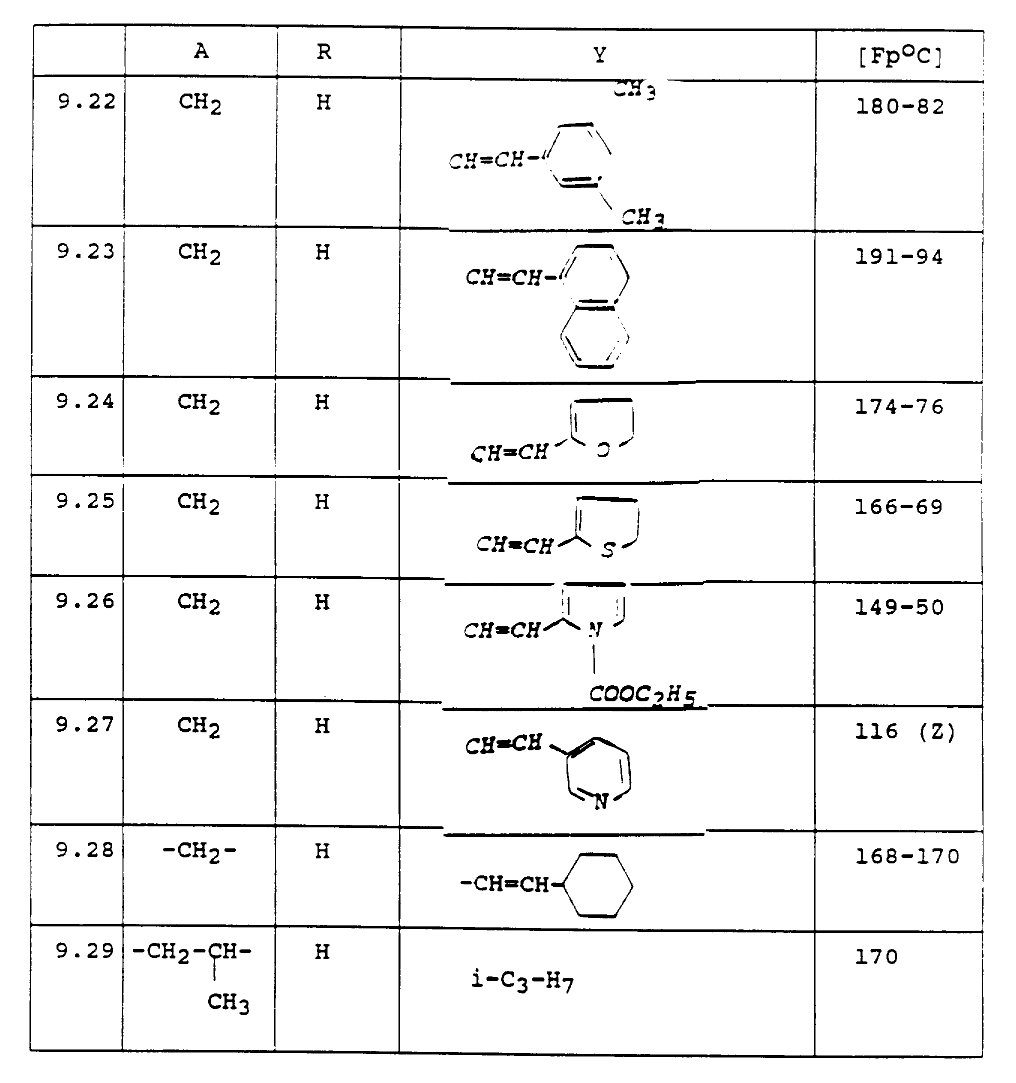

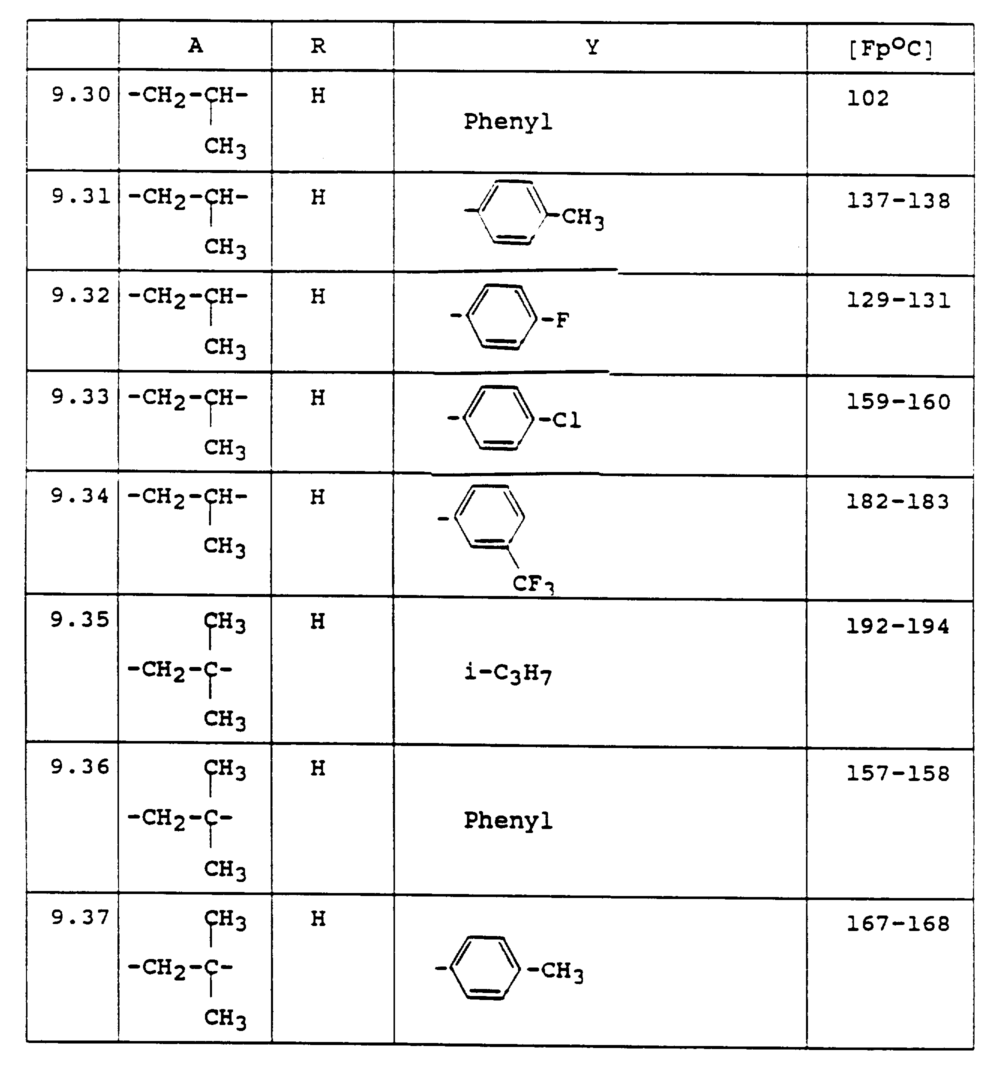

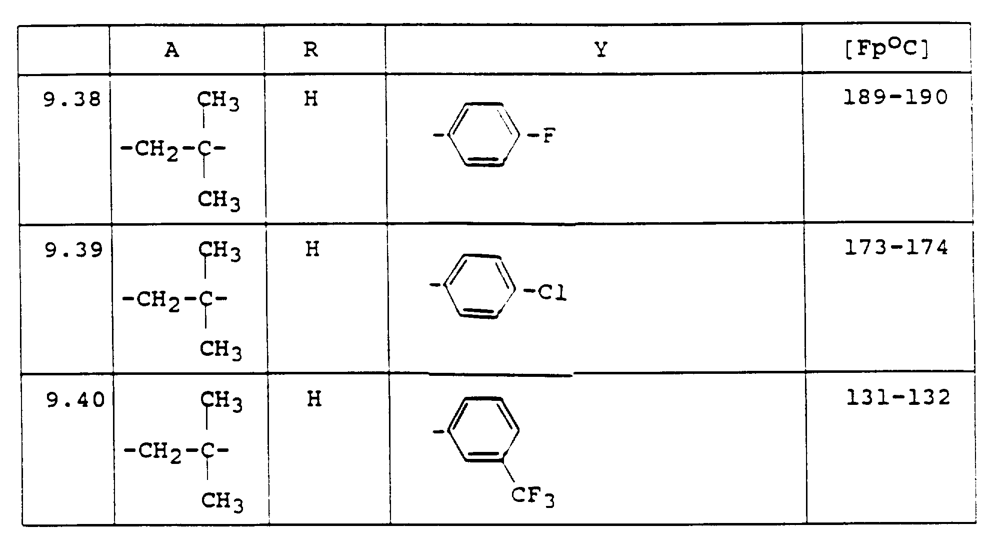

⁺ungesätt. Acrylderivate sind stets (E)-konfiguriert

### Beispiel 10

In analoger Weise wie in Beispiel 1 beschrieben werden die folgenden Verbindungen hergestellt:

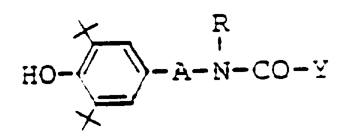

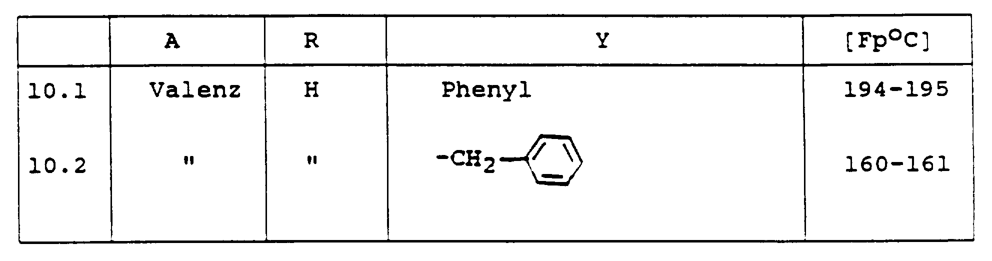

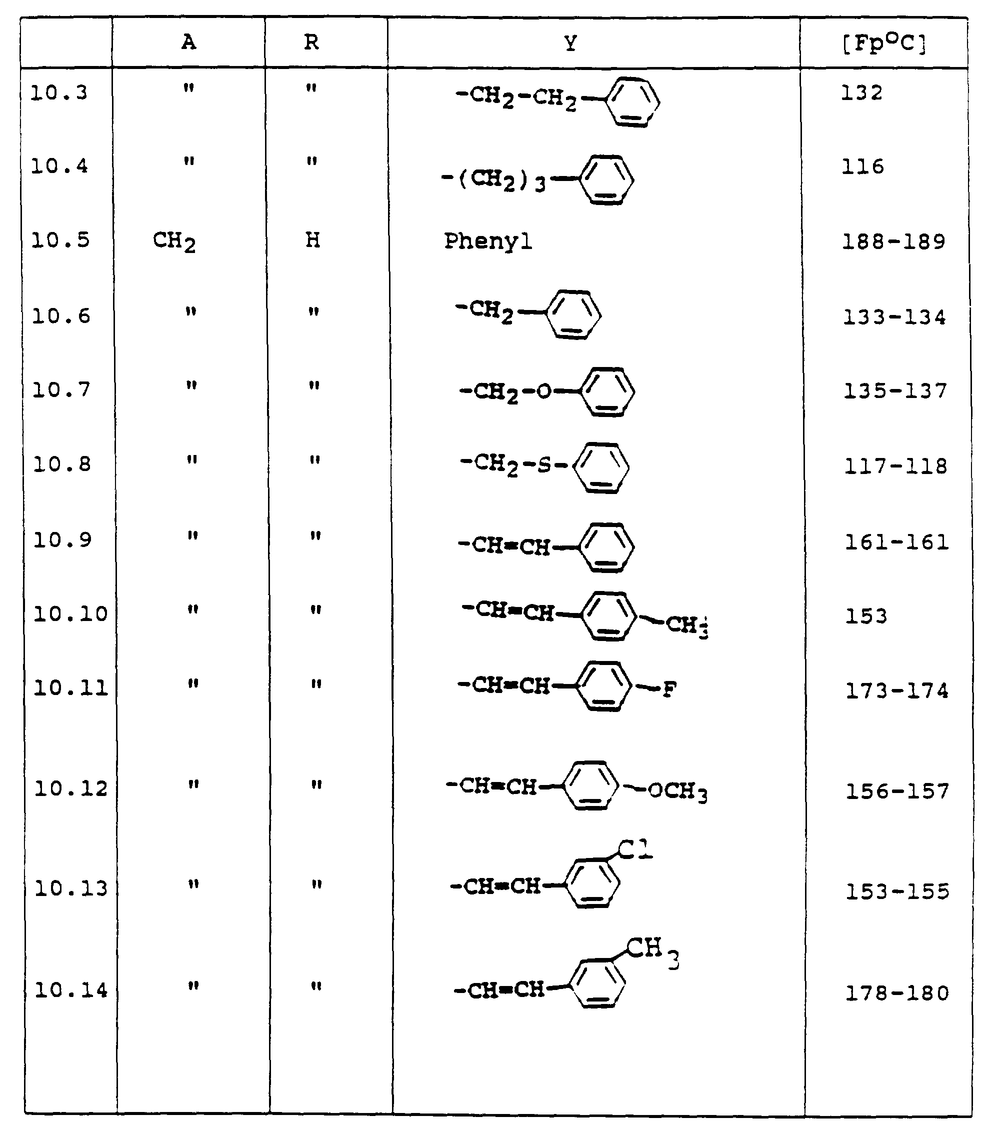

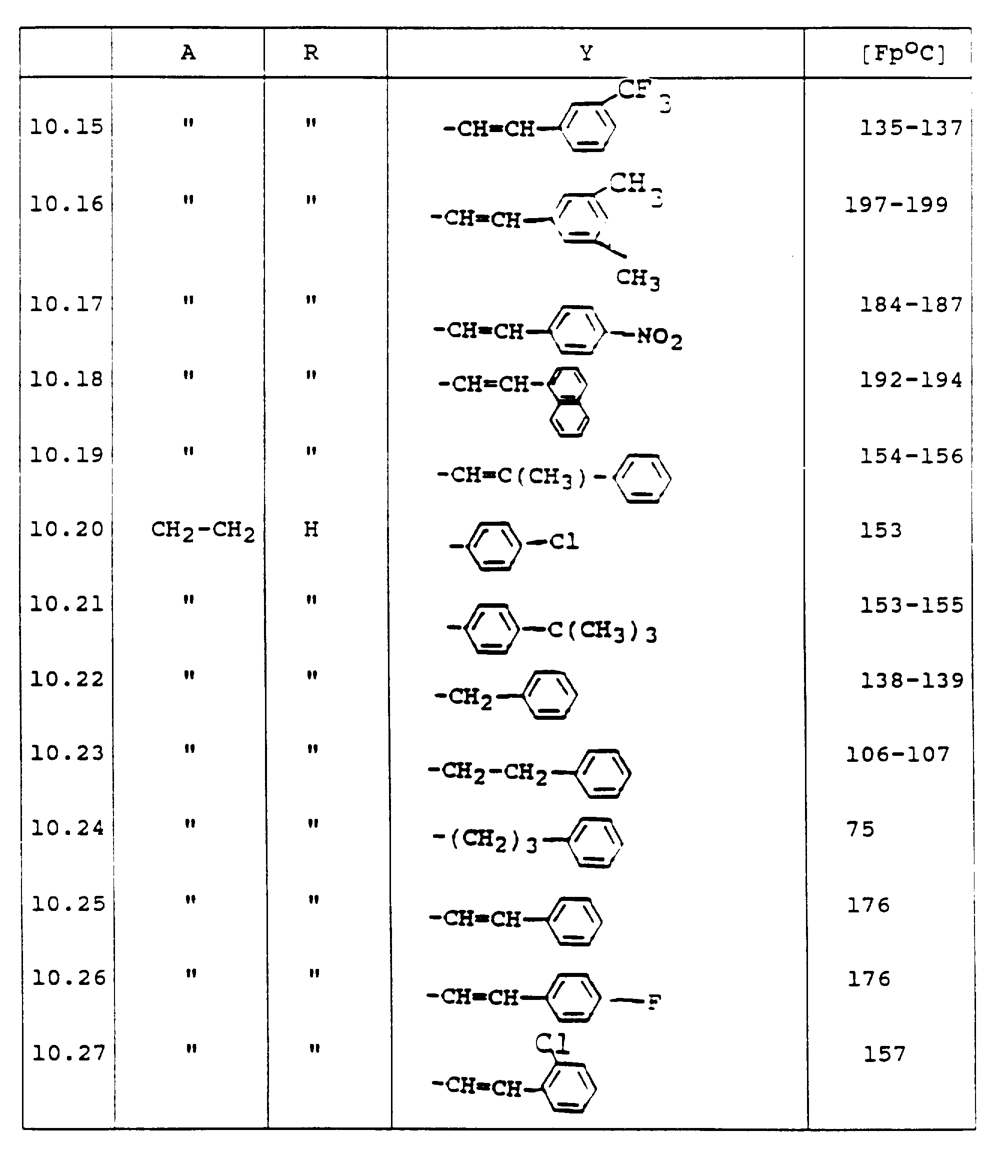

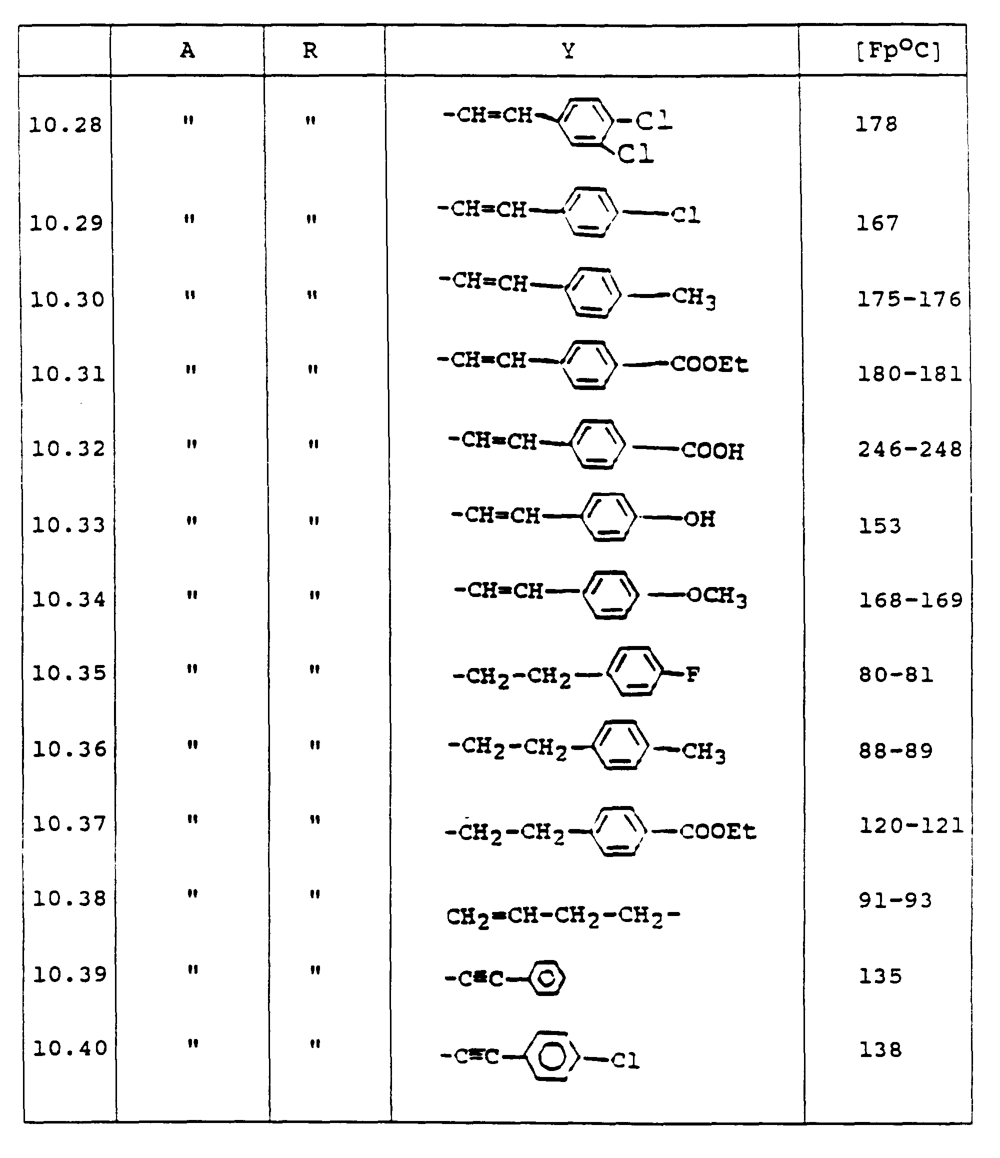

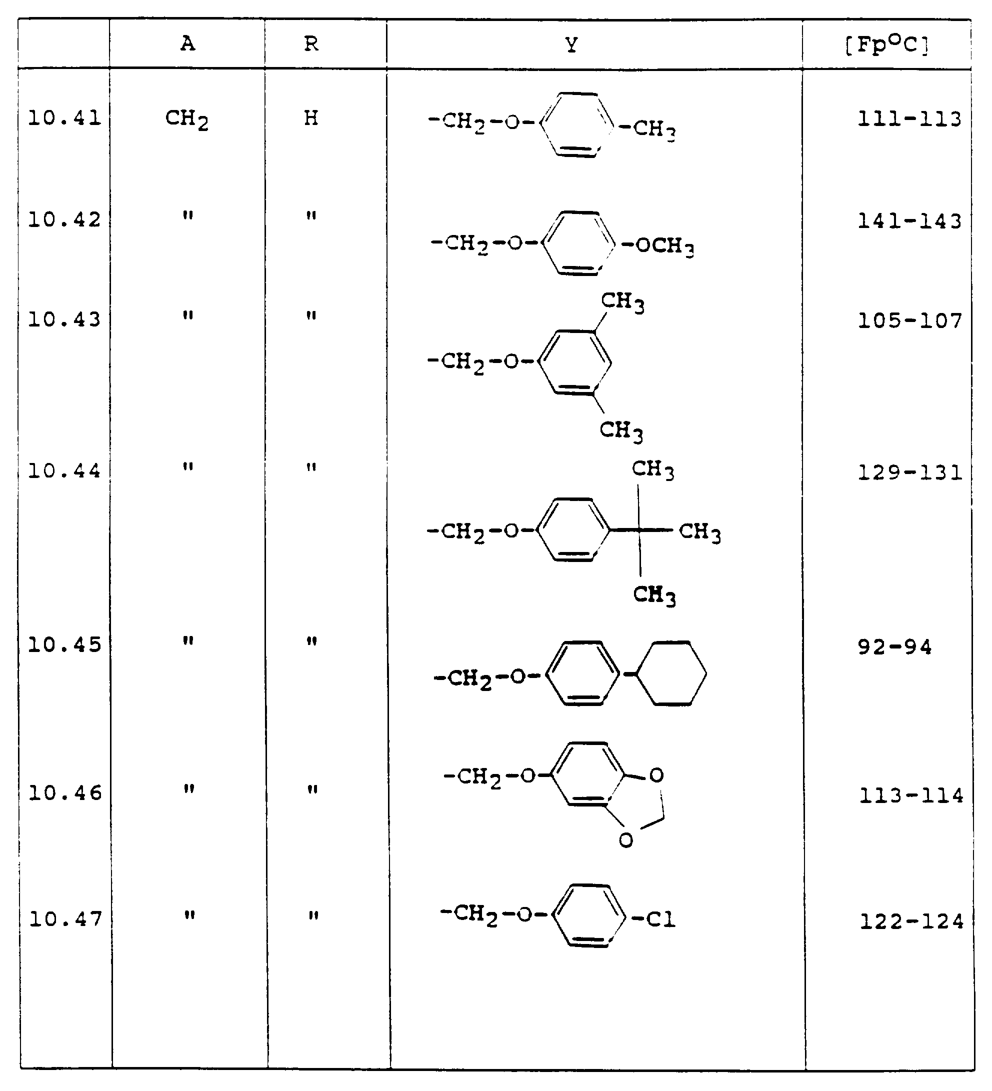

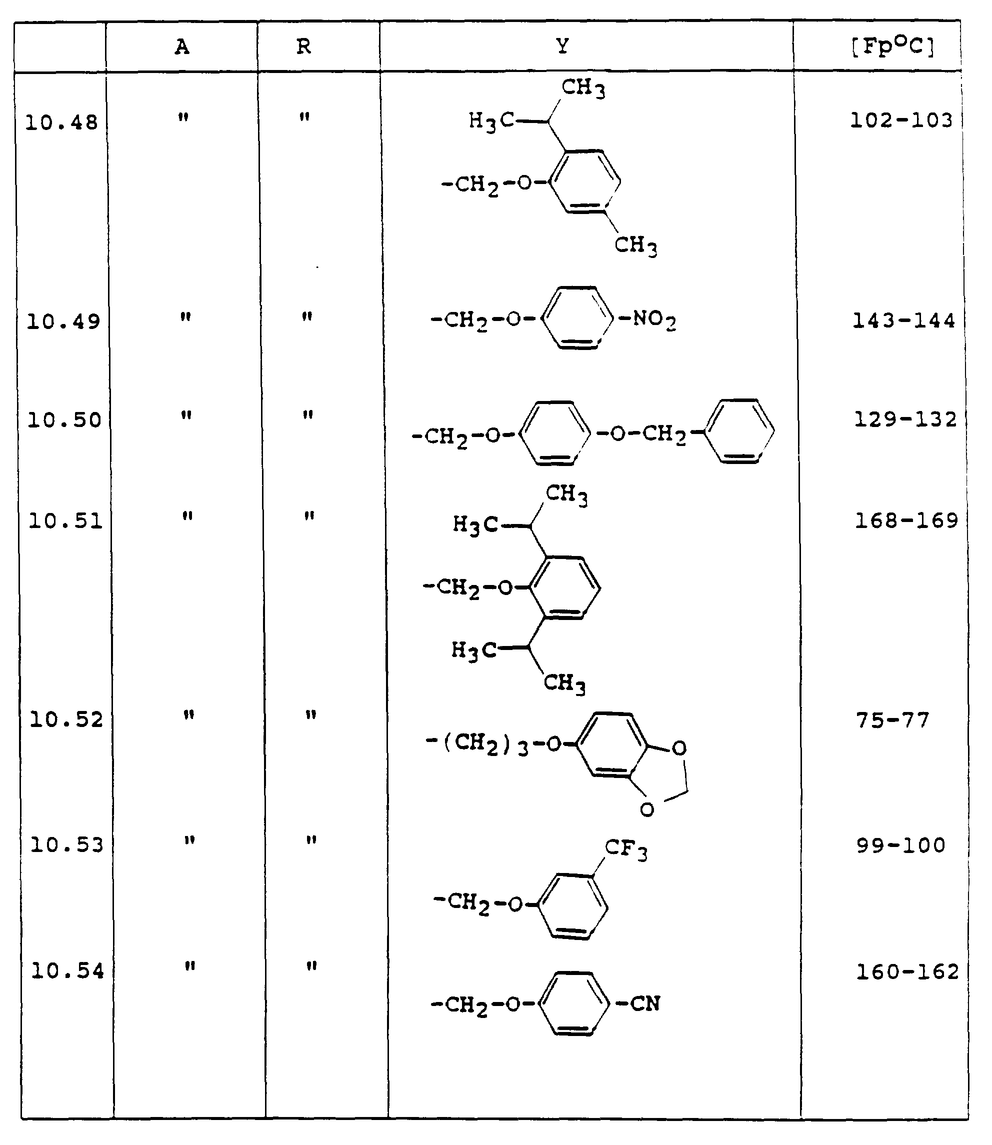

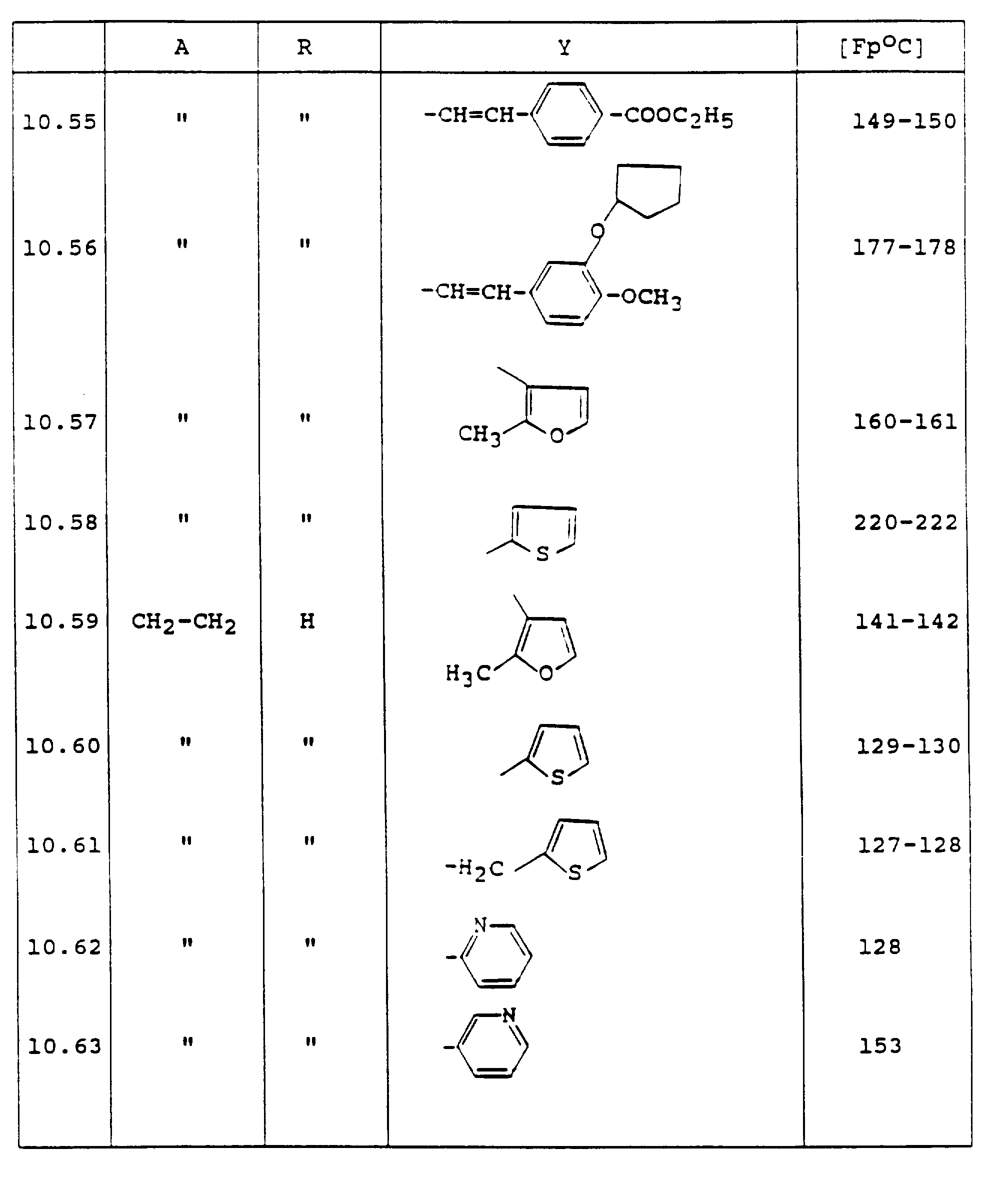

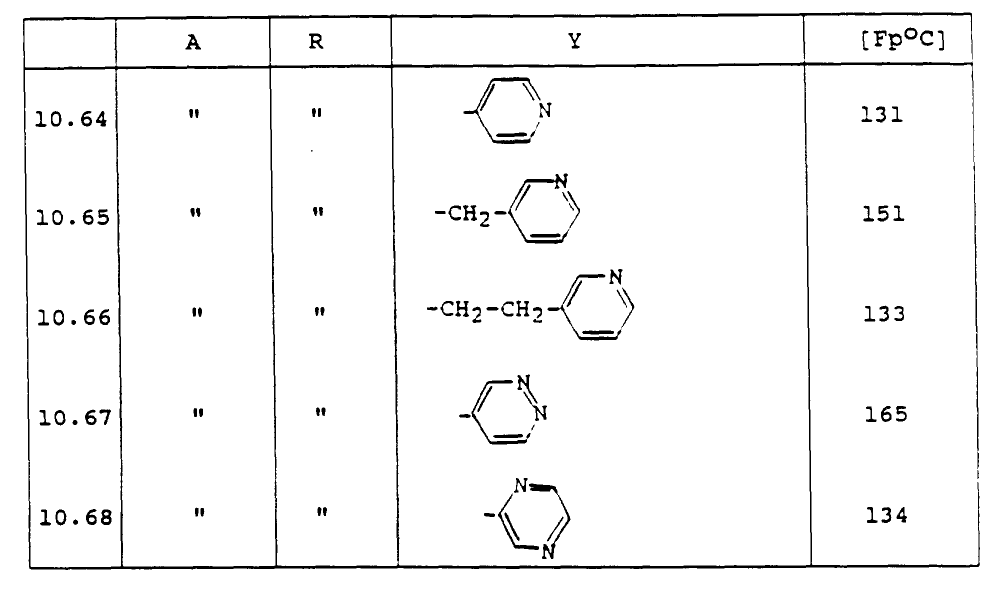

### Beispiel 11

In analoger Weise wie in Beispiel 2 beschreiben werden die folgenden Verbindungen hergestellt:

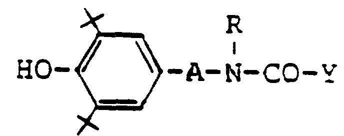

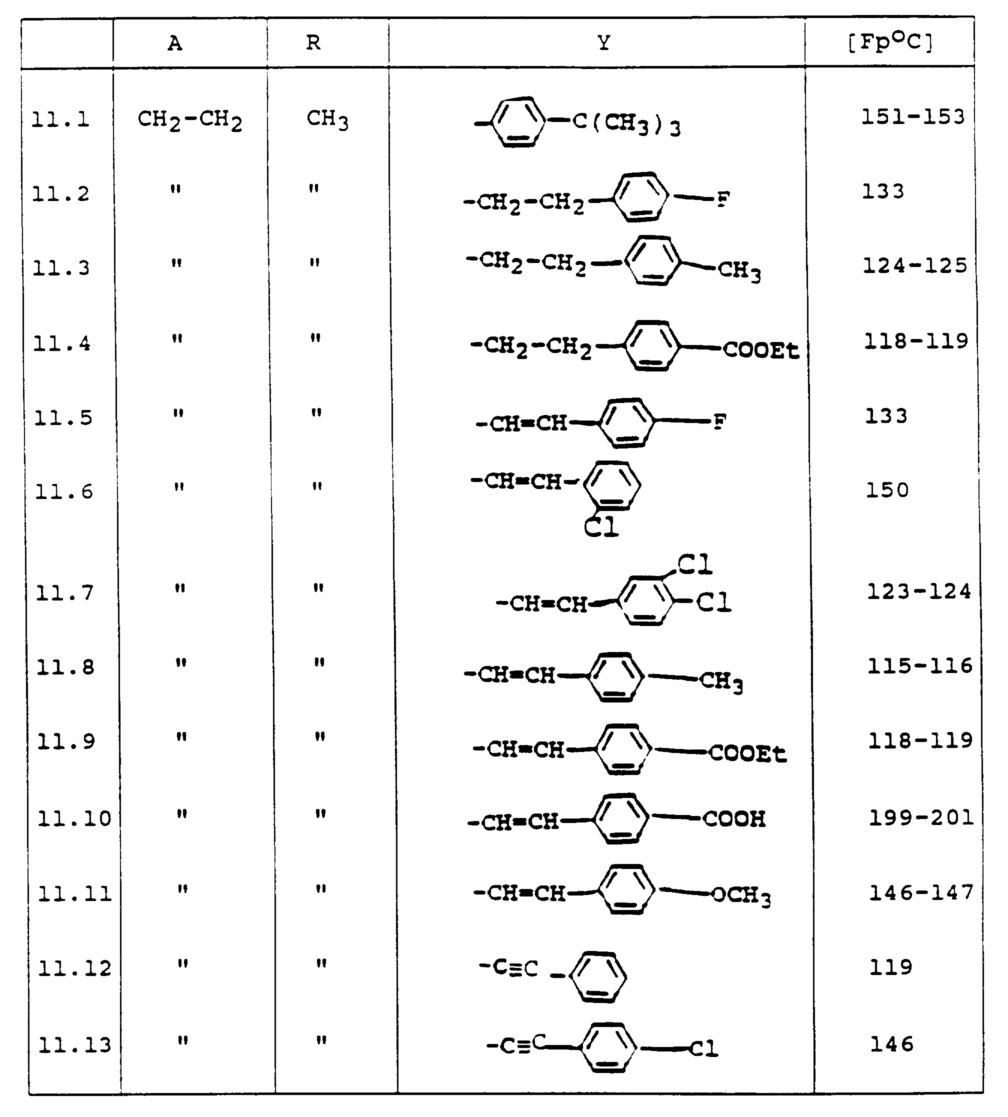

### Beispiel 12

### N-(3,5-Di-tert.butyl-4-hydroxybenzyl)-3-(4-phenoxyessigsäure)acrylamid

5.8 g (0.0124 mol) N-(3,5-Di-tert.butyl-4-hydroxybenzyl)-3-(4-phenoxyessigsäureethylester)acrylamid (Beispiel 6) werden in 50 ml Ethanol und 50 ml Wasser mit 1.06 g (0.019 mol) KOH 24 h bei Raumtemperatur gerührt. Man destilliert den Alkohol ab, säuert mit verd. Salzsäure an und extrahiert mit Essigester. Nach Verdampfen des Essigester kristallisiert das Produkt aus Ether/Isohexan (1:1) aus. Man erhält 1.1 g vom Fp. 125-126°C.

Analog erhält man:
- 12.1: N-(3,5-Di-tert.butyl-4-hydroxybenzyl)-3-(3,4-dihydroxyphenyl)acrylamid fast farblose Kristalle, Fp. 233-235°C aus N-(3,5-Di-tert.butyl-4-hydroxybenzyl-3-(3,4-diacetoxyphenyl)arylamid (Beisp. 5.7)
- 12.2: .N-(3.5-Di-tert.butyl-4-hydroxybenzyl)-3-(4-hydroxycarbonyl-phenyl)acrylamid aus N-3,5-Di-tert.butyl-4-hydroxybenzyl)-3-(4-ethoxycarbonylphenyl)acrylamid (Beisp. 10.55)

### Beispiel 13

### N-(3,5-Di-tert.butyl-4-hydroxybenzyl)-3-(4-pivaloylphenyl)acrylamid

3.8 g (10 mmol) N-(3,5-Di-tert.butyl-4-hydroxybenzyl)-3-(4-hydroxyphenyl)acrylamid (Beisp. 5) werden in 100 ml Tetrahydrofuran gelöst und 2.8 ml (20 mmol) Triethylamin zugegeben. Unter Kühlung werden zwischen 5° und 10°C 1.5 ml (12 mmol) Pivalinsäurechlorid in 5 ml Tetrahydrofuran gelöst, zugetropft. Nach 24 h rühren bei Raumtemperatur wird mit Wasser verdünnt und mit Essigester extrahiert. Der Essigesterrückstand wird über eine Kieselgelsäule (KG 60) mit Isohexan/Essigester gereinigt. Es verbleiben nach Anreiben mit Ether farblose Kristalle vom Fp. 172-173°C.

### Beispiel 14

### N-(3,5-Di-tert.butyl-4-hydroxybenzyl)-3-(4-aminophenyl)acrylamid

4.1 g (10 mmol) N-(3,5-Di-tert.butyl-4-hydroxybenzyl)-3-(4-nitrophenyl)acrylamid (Beisp. 10.17) werden in 250 ml Ethanol gelöst und mit 6.5 g (100 mmol) Zink-Staub versetzt. Innerhalb 1 h werden unter kräftigem Rühren 100 ml 2 molare Salzsäure zugetropft. Nach 3 stündigem Rühren ist die Reaktion beendet. Es wird abgesaugt, das Filtrat abdestilliert und in Essigester und Wasser aufgenommen. Der Essigesterrückstand wird über eine Kieselgelsäule (KG 60) gereinigt. Es werden 1.5 g Produkt vom Fp. 80°C (sintern) nach Anreiben mit Isohexan erhalten.

### Beispiel 15

### N-(3,5-Di-tert.butyl-4-hydroxybenzyl)-4-hydroxy-phenoxyessigsäureamid

1.5 g 3.2 mmol N-(3,5-Di-tert.butyl-4-hydroxybenzyl)-4-benzyloxy-phenoxyessigsäureamid (Beisp. 10.50) werden in 50 ml Methanol gelöst, mit 0.5 g 10proz. Pd-Kohle versetzt und 3 h bei Raumtemperatur und Normaldruck hydriert. Nach Absaugen des Katalysators und Eindampfen des Lösungsmittels werden 1.2 g farblose Kristalle vom Fp. 114-116°C erhalten.

Analog Beispiel 15 erhält man:

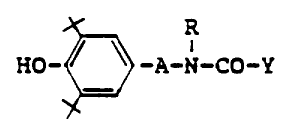

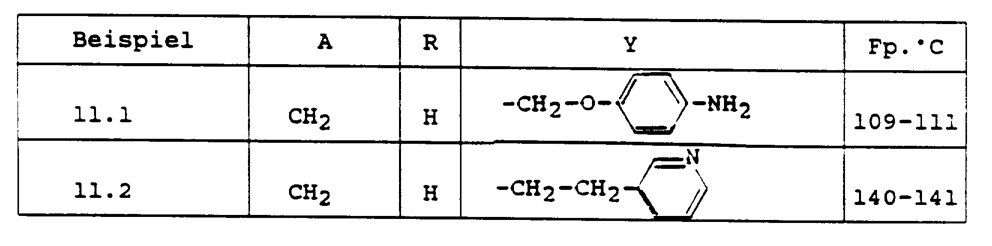

### Beispiel 16

### N-(3,5-Di-tert.butyl-4-hydroxybenzyl)-3-(4-hydroxymethylphenyl)acrylamid

2.7 g 6 mmol N-(3,5-Di-tert.butyl-4-hydroxybenzyl)-3-(4-ethoxycarbonyl-phenyl)acrylamid (Beisp. 10.55) werden mit Li BH₄ (hergestellt aus 1.4 g NaBH₄ 37 mmol und 1.6 g LiCl 37 mmol) in 100 ml Ethylenglycoldimethylether 24 h bei Raumtemperatur und anschließend 4 h bei 50°C gerührt. Der Reduktionskomplex wird mit Wasser und 2 mol Schwefelsäure zersetzt und das Produkt mit Essigester extrahiert. Nach Einengen der Essigesterphase und Anreiben mit Ether/Isohexan werden 1.1 g farblose Kristalle vom Fp. 177-179°C erhalten.

## Patentansprüche

1. Verbindungen der Formel I 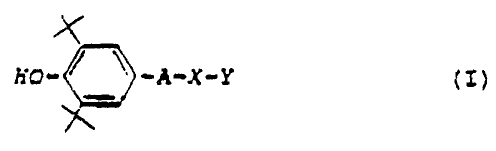 in der
A eine gerade oder eine verzweigte Alkylkette mit 1 bis 5 C-Atomen
X eine -NR-CO- (Amid) oder eine -NR-CO-NR-(Harnstoff) Gruppe, in der R ein Wasserstoffatom oder einen C₁-C₄-Alkylrest darstellt,
und
Y eine geradkettige oder verzweigte, gesättigte oder ungesättigte Kohlenwasserstoffkette mit 1 bis 6 C-Atomen, die durch einen Aryl-, Hetaryl- Aryloxy-, oder Arylthio-Rest substituiert sein kann, einen C₃-C₆-Cycloalkyl-, oder einen Arylrest darstellt, wobei der betreffende Aryl- bzw. Hetarylrest jeweils ein bis dreifach in allen möglichen Positionen am Ring durch CN, Hydroxymethyl, Methylendioxy-, Halogen, Trifluormethyl, C₁-C₄-Alkyl, Amino, C₁-C₄-Acylamino, Di-(C₁-C₄)-alkylamino, C₁-C₄-Alkoxy, Carboxyl, Oxyessigsäureethylester oder Nitro substituiert sein kann,
bedeuten,
mit der Maßgabe, daß Y nur unsubstituiertes Phenyl bedeutet, wenn A eine andere Bedeutung als -CH₂- oder -CH₂-CH₂- hat,
sowie deren pharmakologische unbedenkliche Salze.

2. Verfahren zur Herstellung von Verbindungen der Formel I 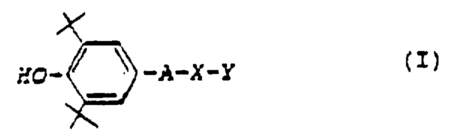 in der
A eine gerade oder eine verzweigte Alkylkette mit 1 bis 5 C-Atomen
X eine -NR-CO- (Amid) oder eine -NR-CO-NR-(Harnstoff) Gruppe, in der R ein Wasserstoffatom oder einen C₁-C₄-Alkylrest darstellt,
und
Y eine geradkettige oder verzweigte, gesättigte oder ungesättigte Kohlenwasserstoffkette mit 1 bis 6 C-Atomen, die durch einen Aryl-, Hetaryl- Aryloxy-, oder Arylthio-Rest substituiert sein kann, einen C₃-C₆-Cycloalkyl-, oder einen Arylrest darstellt, wobei der betreffende Aryl- bzw. Hetarylrest jeweils ein bis dreifach in allen möglichen Positionen am Ring durch CN, Hydroxymethyl, Methylendioxy-, Halogen, Trifluormethyl, C₁-C₄-Alkyl, Amino, C₁-C₄-Acylamino, Di-(C₁-C₄)-alkylamino, C₁-C₄ -Alkoxy, Carboxyl, Oxyessigsäureethylester oder Nitro substituiert sein kann,
bedeuten,
mit der Maßgabe, das Y nur unsubstituiertes Phenyl bedeutet, wenn A eine andere Bedeutung als -CH₂- oder -CH₂-CH₂- hat,
sowie deren pharmakologische unbedenkliche Salze, dadurch gekennzeichnet, daß man in an sich bekannter Weise
a) für den Fall, daß X eine -CO-NR-Gruppe darstellt, eine Verbindung der Formel II 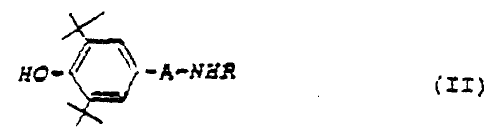 in der A und R die angegebene Bedeutung haben, mit einer Verbindung der Formel III 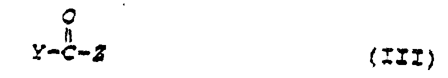 in der Y die angegebene Bedeutung hat und Z Hydroxyl, Halogen, C₁-C₄-Alkoxy oder Azid darstellt,
umsetzt,
und
b) für den Fall, daß X eine -NR-CO-NR-Gruppe darstellt, eine Verbindung der Formel II 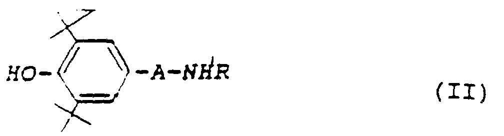 in der A und R die angegebene Bedeutung haben,
mit einem Isocyanat der Formel IV umsetzt
O=C=N-Y (IV)
in der Y die angegebene Bedeutung hat,
und anschließend gewünschtenfalls die erhaltenen Verbindungen der Formel I durch geeignete Maßnahmen in andere Verbindungen der Formel I umwandelt oder die Verbindungen in pharmakologisch unbedenkliche Salze überführt.

3. Arzneimittel, enthaltend eine Verbindung gemäß Anspruch 1, neben üblichen Träger- und Hilfsstoffen.

4. Verwendung von Verbindungen gemäß Anspruch 1 zur Herstellung von Arzneimitteln zur Behandlung von Stoffwechselerkrankungen.

## Claims

1. Compounds of the formula I 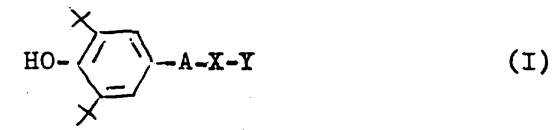 in which A signifies a straight or branched alkyl chain with 1 to 5 C-atoms, X an -NR-CO- (amide) or an -NR-CO-NR- (urea) group in which R respresents a hydrogen atom or a C₁-C₄-alkyl radical and Y a straight-chained or branched, saturated or unsaturated hydrocarbon chain with 1 to 6 C-atoms, which can be substituted by an aryl, hetaryl, aryloxy or arylthio radical, a C₃-C₆-cycloalkyl or an aryl radical, whereby the aryl or hetaryl radical in question can, in each case, be substituted one to three times in all possible positions on the ring by CN, hydroxymethyl, methylenedioxy, halogen, trifluoromethyl, C₁-C₄-alkyl, amino, C₄-C₄-acylamino, di-(C₁-C₄)-alkylamino, C₁-C₄-alkoxy, carboxyl, oxyacetic acid ethyl ester or nitro, with the proviso that Y only signifies unsubstituted phenyl when A has a meaning other than -CH₂- or -CH₂ -CH₂-, as well as their pharmacologically acceptable salts.

2. Process for the preparation of compounds of the formula I 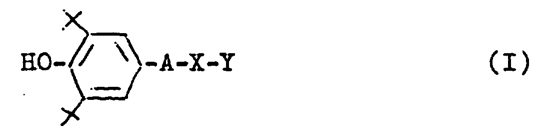 in which A signifies a straight or branched alkyl chain with 1 to 5 C-atoms, X an -NR-CO- (amide) or an -NR-CO-NR- (urea) group in which R represents a hydrogen atom or a C₁-C₄-alkyl radical and Y a straight-chained or branched, saturated or unsaturated hydrocarbon chain with 1 to 6 C-atoms, which can be substituted by an aryl, hetaryl, aryloxy or arylthio radical, a C₃-C₆-cycloalkyl or an aryl radical, whereby the aryl and hetaryl radicals in question can, in each case, be substituted one to three times in all possible positions of the ring by CN, hydroxymethyl, methylenedioxy, halogen, trifluoromethyl, C₁-C₄-alkyl, amino, C₁-C₄-acylamino, di-(C₁-C₄)-alkylamino, C₁-C₄-alkoxy, carboxyl, oxyacetic acid ethyl ester or nitro, with the proviso that Y only signifies unsubstituted phenyl when A has a meaning other than -CH₂- or -CH₂-CH₂-, as well as of their pharmacologically acceptable salts, characterised in that, in per se known manner, one
a) for the case that X represents a -CO-NR- group, reacts a compound of the formula II 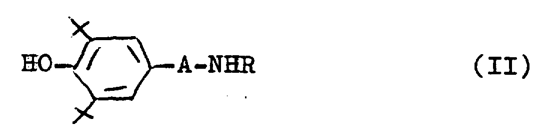 in which A and R have the given meaning, with a compound of the formula III 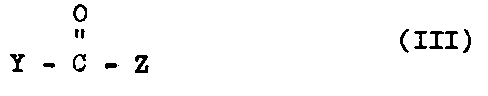 in which Y has the given meaning and Z represents hydroxyl, halogen, C₁-C₄-alkoxy or azide; and
b) for the case that X represents an -NR-CO-NR- group, reacts a compound of the formula II 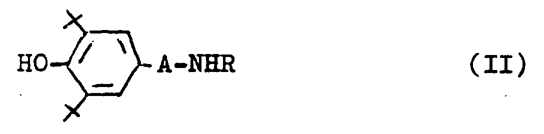 in which A and R have the given meaning, with an isocyanate of the formula IV
O=C=N-Y (IV)
in which Y has the given meaning, and subsequently, if desired, converts the compounds obtained of the formula I by suitable measures into other compounds of the formula I or converts the compounds into pharmacologically acceptable salts.

3. Medicaments containing a compound according to claim 1, besides usual carrier and adjuvant materials.

4. Use of coompounds according to claim 1 for the preparation of medicaments for the treatment of metabolic diseases.

## Revendications

1. Composés de formule I 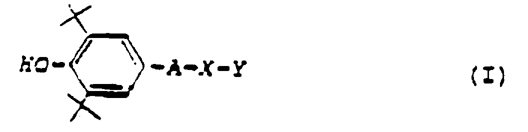 dans laquelle
A représente une chaîne alkyle linéaire ou ramifiée ayant 1 à 5 atomes de C,
X représente un groupe -NR-CO- (amide) ou un groupe -NR-CO-NR- (urée) dans lequel R représente un atome d'hydrogène ou un reste alkyle en C₁-C₄,
et
Y représente une chaîne hydrocarbonée linéaire ou ramifiée, saturée ou insaturée, ayant de 1 à 6 atomes de C, qui peut porter comme substituant un reste aryle, hétéroaryle, aryloxy ou arylthio ; un reste cycloalkyle en C₃-C₆ ou un reste aryle, le reste aryle ou hétéroaryle concerné pouvant toujours porter, dans toutes les positions possibles du cycle, un à trois substituants CN, hydroxyméthyle, méthylènedioxy, halogéno, trifluorométhyle, alkyle en C₁-C₄, amino, (acyle en C₁-C₄)-amino, di(alkyle en C₁-C₄)amino, alcoxy en C₁-C₄, carboxyle, oxyacétate d'éthyle ou nitro,
étant entendu que Y ne peut représenter qu'un groupe phényle non substitué lorsque A a une autre signification que -CH₂- ou -CH₂-CH₂-,
ainsi que leurs sels acceptables en pharmacie.

2. Procédé de préparation de composés de formule I 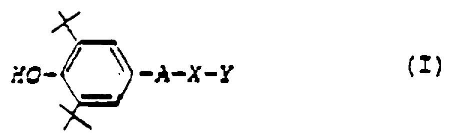 dans laquelle
A représente une chaîne alkyle linéaire ou ramifiée ayant 1 à 5 atomes de C,
X représente un groupe -NR-CO- (amide) ou un groupe -NR-CO-NR- (urée) dans lequel R représente un atome d'hydrogène ou un reste alkyle en C₁-C₄,
et
Y représente une chaîne hydrocarbonée linéaire ou ramifiée, saturée ou insaturée, ayant de 1 à 6 atomes de C, qui peut porter comme substituant un reste aryle, hétéroaryle, aryloxy ou arylthio ; un reste cycloalkyle en C₃-C₆ ou un reste aryle, le reste aryle ou hétéroaryle concerné pouvant toujours porter, dans toutes les positions possibles du cycle, un à trois substituants CN, hydroxyméthyle, méthylènedioxy, halogéno, trifluorométhyle, alkyle en C₁-C₄, amino, (acyle en C₁-C₄)-amino, di(alkyle en C₁-C₄)amino, alcoxy en C₁-C₄, carboxyle, oxyacétate d'éthyle ou nitro,
étant entendu que Y ne peut représenter qu'un groupe phényle non substitué lorsque A a une autre signification que -CH₂- ou -CH₂-CH₂-,
ainsi que leurs sels acceptables en pharmacie, caractérisé par le fait que, de manière connue en soi, on fait réagir
a) dans le cas où X représente un groupe -CO-NR-, un composé de formule Il 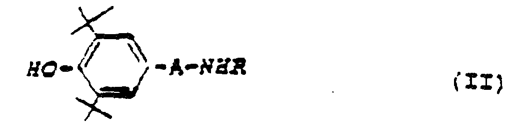 dans laquelle A et R ont les significations mentionnées, avec un composé de formule III 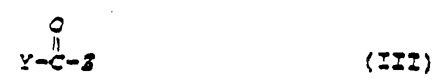 dans laquelle Y a la signification mentionnée et Z représente un groupe hydroxyle, halogéno, alcoxy en C₁-C₄ ou azoture,
et
b) dans le cas où X représente un groupe -NR-CO-NR-, un composé de formule Il 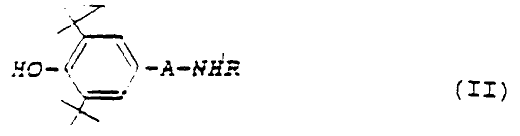 dans laquelle A et R ont les significations mentionnées,
avec un isocyanate de formule IV
O=C=N-Y ( IV)
dans laquelle Y a la signification mentionnée,
et ensuite, on transforme éventuellement les composés de formule obtenus, par des opérations appropriées, en d'autres composés de formule ou en leurs sels acceptables en pharmacie.

3. Médicament contenant un composé de formule I, en plus de véhicules et adjuvants usuels.

4. Utilisation de composés selon la revendication 1, pour la préparation de médicaments destinés au traitement de troubles du métabolisme.
